# EUROPEAN PATENT APPLICATION

(11) **EP 4 840 464 A1**
(43) Date of publication of application: **18.11.2026**
(21) Application number: 25738389.3
(22) Date of filing: 07.01.2025
(51) Int. Cl.: A61K 45/06, A61P 35/00

(54) **FAK INHIBITOR AND PAN-RAS INHIBITOR**

(30) Priority: 08.01.2024 CN 202410023478; 18.12.2024 CN 202411875224
(71) Applicant: Inxmed (Nanjing) Co., Ltd., Nanjing, Jiangsu 210000 (CN)
(72) Inventor: ZHANG, Baoyuan, Nanjing, Jiangsu 210000 (CN); GAO, Jiaming, Nanjing, Jiangsu 210000 (CN); LIU, Xuebin, Nanjing, Jiangsu 210000 (CN); WANG, Zaiqi, Nanjing, Jiangsu 210000 (CN)
(74) Representative: D Young & Co LLP
(86) International application number: PCT/CN2025/070950
(87) International publication number: WO 2025/148858

(57) **Abstract**

The present disclosure relates to combining an FAK inhibitor and a Pan-RAS inhibitor to treat tumor.

## Description

### TECHNICAL FIELD

The present disclosure belongs to the field of pharmaceutical chemistry. Specifically, the present disclosure relates to the combination of focal adhesion kinase (FAK) inhibitors and Pan-RAS inhibitors for the treatment of tumors.

### BACKGROUND

RAS is the oncogene with the highest mutation frequency, comprising three main subtypes: KRAS, NRAS, and HRAS. Approximately 30 % of cancers carry the RAS mutation, wherein KRAS has the highest mutation rate in cancer species such as lung cancer, pancreatic cancer and colorectal cancer, NRAS mutation mainly appears in melanoma and leukaemia, and HRAS has a lower mutation rate and mainly appears in cancer species such as head and neck cancer. RAS is considered to be undruggable by both academia and the industry for a long period of time after the RAS gene mutation was found to cause cancer, until the discovery of the KRAS G12C targeted pocket in 2013. Targeting this pocket allows the KRAS to remain in an inactive state, thereby inhibiting KRAS activity and producing an anti-tumor effect. Based on this principle, multiple leading pharmaceutical companies have developed KRAS G12C inhibitors, among which the most advanced, Sotorasib and Adagrasib, have been marketed primarily for the treatment of non-small cell lung cancer (NSCLC). Meanwhile, inhibitors targeting other KRAS mutation sites, such as KRAS G12D, have also progressed rapidly, wherein MRTX1133 and HRS-4642 have entered human clinical trials, and the data will gradually mature. In recent years, the concept of using a single inhibitor to target multiple RAS mutations at the same time has become increasingly noticeable. Pan RAS inhibitors, represented by RMC6236 from Revolution Medicine, have also entered clinical trials and can be used to treat tumors targeting different RAS mutations such as KRAS, NRAS, and HRAS, and the preliminary results from clinical trials are encouraging.

Despite the rapid progress, the general problem of targeting inhibitors is the rapid production of drug resistance, thereby affecting the persistence of the drug effect. Therefore, there is still a need for new therapeutic regimens for tumors with different RAS mutations.

### SUMMARY

The inventors have found that the combination of a FAK inhibitor and a Pan-RAS inhibitor produces a superior effect. This regimen will address the issues of insufficient efficacy and primary resistance of Pan-RAS inhibitors, and will also significantly enhance the response of patients with cancer carrying RAS mutations to such inhibitors.

In one aspect, the present disclosure provides the use of an FAK inhibitor and a Pan-RAS inhibitor in the manufacture of a medicament for treating a tumor in a subject.

In another aspect, the present disclosure provides a pharmaceutical combination product of an FAK inhibitor and a Pan-RAS inhibitor for treating a tumor in a subject.

In another aspect, the present disclosure provides a method for treating a tumor, the method comprising administering a therapeutically effective amount of a FAK inhibitor and a Pan-RAS inhibitor to a subject in need thereof.

In another aspect, the present disclosure provides a kit or pharmaceutical composition for treating a tumor, comprising:
(a) a FAK inhibitor; and
(b) a Pan-RAS inhibitor.

In another aspect, the present disclosure provides the use of a FAK inhibitor and a Pan-RAS inhibitor in the manufacture of a combination medicament for treating a tumor.

In another aspect, the present disclosure provides the use of a FAK inhibitor in the manufacture of a combination medicament for use in combination with a Pan-RAS inhibitor to treat a tumor.

In another aspect, the present disclosure provides the use of a Pan-RAS inhibitor in the manufacture of a combination medicament for use in combination with a FAK inhibitor to treat a tumor.

In another aspect, the present disclosure provides a kit comprising: a FAK inhibitor; and instructions indicating that the FAK inhibitor can be used in combination with a Pan-RAS inhibitor for treating a tumor.

In another aspect, the present disclosure provides a kit comprising: a Pan-RAS inhibitor; and instructions indicating that the Pan-RAS inhibitor can be used in combination with a FAK inhibitor for treating a tumor.

Optionally, the FAK inhibitor and the Pan-RAS inhibitor are administered to the subject simultaneously or sequentially.

The FAK inhibitor is, for example, IN10018, Defactinib, AMP945, a deuterated compound thereof, or a pharmaceutically acceptable salt thereof; alternatively, IN10018, Defactinib, AMP945, deuterated compound 1 of Defactinib (CAS No. 2384121-03-1), deuterated compound 2 of Defactinib (CAS No. 2384120-99-2), or a pharmaceutically acceptable salt thereof; more alternatively, IN10018, Defactinib, AMP945, or a pharmaceutically acceptable salt thereof; and even more alternatively, IN10018 or a pharmaceutically acceptable salt thereof.

The Pan-RAS inhibitor is, for example, RMC6236, Pan-RAS-IN-1, or ADT-007.

Optionally, the tumor is a tumor with RAS mutation.

The tumor is, for example, bladder cancer, breast cancer, cervical cancer, colon cancer (including colorectal cancer), esophageal cancer, esophageal squamous cell carcinoma, head and neck cancer, liver cancer, lung cancer (including small cell lung cancer and non-small cell lung cancer), melanoma, myeloma, rhabdomyosarcoma, inflammatory myofibroblastoma, neuroblastoma, pancreatic cancer, prostate cancer, kidney cancer, renal cell carcinoma, fibrosarcoma (including osteosarcoma), skin cancer (including squamous cell carcinoma), gastric cancer, testicular cancer, thyroid cancer, uterine cancer, mesothelioma, bile duct carcinoma, leiomyosarcoma, liposarcoma, nasopharyngeal carcinoma, neuroendocrine carcinoma, ovarian cancer, salivary gland cancer, metastatic tumors caused by spindle cell carcinoma, anaplastic large cell lymphoma, undifferentiated thyroid carcinoma, non-Hodgkin's lymphoma, Hodgkin's lymphoma, glioma, or hematologic malignancies such as acute myeloid leukemia (AML), acute lymphoblastic leukemia (ALL), diffuse large B-cell lymphoma (DLBCL), follicular lymphoma (FL), chronic lymphocytic leukemia (CLL), or chronic myeloid leukemia (CML); alternatively, the tumor is fibrosarcoma, gastric cancer, lung cancer, breast cancer, glioma, esophageal cancer, pancreatic cancer, head and neck cancer, ovarian cancer, or colon cancer (including colorectal cancer); more alternatively, the tumor is colon cancer (including colorectal cancer), lung cancer, pancreatic cancer, or fibrosarcoma; even more alternatively, the tumor is colon cancer (including colorectal cancer) or fibrosarcoma.

The pharmaceutically acceptable salt of IN10018 is, for example, a tartrate salt.

The pharmaceutically acceptable salt of AMP945 is, for example, a tartrate salt.

The pharmaceutically acceptable salt of Defactinib is, for example, a hydrochloride salt.

The use, pharmaceutical combination product, method, kit or pharmaceutical composition, including a therapeutic agent.

The therapeutic agent is an immune checkpoint inhibitor.

The immune checkpoint inhibitor is PD-1 or PD-L1 inhibitor, especially PD-1 or PD-L1 antibody inhibitor.

### BRIEF DESCRIPTION OF DRAWINGS

To illustrate the technical solution of the examples of the present disclosure more clearly, the drawings of the examples will be briefly described below, and it will be apparent that the drawings described below only relate to some examples of the present disclosure, and are not intended to limit the present disclosure.
Fig. 1 shows the effect of the combination of RMC6236 and IN10018 on the proliferation of HT1080 cells in Example 1.
Fig. 2 shows the effect of the combination of RMC6236 and AMP945 on the proliferation of HT1080 cells in Example 1.
Fig. 3 shows the effect of the combination of RMC6236 and Defactinib on the proliferation of HT1080 cells in Example 1.
Fig. 4 shows the effect of the combination of RMC6236 and IN10018 on the proliferation of CT26 cells in Example 2.
Fig. 5 shows the effect of the combination of RMC6236 and AMP945 on the proliferation of CT26 cells in Example 2.
Fig. 6 shows the effect of the combination of RMC6236 and Defactinib on the proliferation of CT26 cells in Example 2.
Fig. 7 shows the effect of RMC6236 on CT26 cell proliferation when the FAK target is silent in Example 3.
Figs. 8-1 and 8-2 show the in vitro proliferation inhibition of IN10018 alone on RAS mutant cell lines in Example 4. Fig. 8-1 shows the in vitro proliferation inhibition of Capan1 cells; Fig. 8-2 shows the in vitro proliferation inhibition of NCI-H358 cells. Data are expressed as mean ± SEM.
Figs. 9-1 and 9-2 show the in vitro proliferation inhibition of RAC6236 alone and in combination with IN10018 on RAS mutant cell lines in Example 4. Fig. 9-1 shows the in vitro proliferation inhibition of Capan1 cells; Fig. 9-2 shows the in vitro proliferation inhibition of NCI-H358 cells. Data are expressed as mean ± SEM.
Figs. 10-1 and 10-2 show the in vitro proliferation inhibition of Pan-RAS-IN-1 alone and in combination with IN10018 on RAS mutant cell lines in Example 4. Fig. 10-1 shows the in vitro proliferation inhibition of Capan1 cells; Fig. 10-2 shows the in vitro proliferation inhibition of NCI-H358 cells. Data are expressed as mean ± SEM.
Figs. 11-1 and 11-2 show the in vitro proliferation inhibition of ADT-007 alone and in combination with IN10018 on RAS mutant cell lines in Example 4. Fig. 11-1 shows the in vitro proliferation inhibition of Capan1 cells; Fig. 11-2 shows the in vitro proliferation inhibition of NCI-H358 cells. Data are expressed as mean ± SEM.
Fig. 12 shows the tumor growth curve after administration in CT26 cell subcutaneous xenograft model in Example 5. Data are expressed as mean ± SEM.
Fig. 13 shows the tumor growth curve after administration in Capan1 cell subcutaneous xenograft model in Example 6. Data are expressed as mean ± SEM.

### DETAILED DESCRIPTION

To make the objects, technical solutions and advantages of the embodiments of the present disclosure clearer, the technical solutions of the embodiments of the present disclosure will be clearly and fully described below by reference to the accompany drawings of the embodiments of the present disclosure. It is obvious that the described embodiments are just some, but not all of the embodiments of the present disclosure. Based on the described embodiments of the present disclosure, all other embodiments obtained by those of ordinary skill in the art without creative effort will fall within the scope of protection of the present disclosure.

The present disclosure can be implemented in other specific forms without departing from the spirit of the present disclosure. It should be understood that any and all embodiments of the present disclosure can be combined with the technical feature(s) of any other embodiment(s) to obtain alternative embodiments on the premise of no conflict. The invention encompasses such combination to obtain alternative embodiments.

All the publications and patents mentioned in the present disclosure are hereby incorporated by reference in their entirety. If the use or terminology used in any publication and patent incorporated by reference conflicts with the use or terminology used in the present disclosure, the use and terminology of the present disclosure shall prevail.

The section headings used herein are used for organizational purposes only and are not to be construed as limiting the subject matter described.

Unless otherwise specified, all technical and scientific terms used herein have the same meaning as those commonly used in the art to which the claimed subject matter belongs. If there are multiple definitions for a term, the definition herein shall prevail.

The following terms and symbols used in the present application have the meanings described below, unless otherwise specified in the context.

Unless in the working examples or otherwise indicated, all numbers stating quantitative properties such as dosage in the specification and claims should be understood as modified by the term "about" in all instances. It should also be understood that any numerical range enumerated in the present application is intended to include all sub-ranges within that range and any combination of the endpoints of that range or sub-ranges.

The "comprise/comprising," "include/including," or "contain/containing," or similar terms are intended to specify that the elements stated before these terms encompass the elements and equivalents thereof listed after these terms, but do not preclude the elements not recited. The term "comprise," "include (contain)" used herein can be in open, semi-close, and close forms. In other words, the term also comprises "consisting essentially of" or "consisting of."

As used herein, "FAK inhibitor" refers to an effective inhibitor of FAK that is suitable for mammals, particularly humans. In some embodiments, the FAK inhibitor is IN10018 (CAS No.: 1227948-82-4), Defactinib (CAS No.: 1073154-85-4 ), AMP945 (CAS No.: 1393653-34-3 ), a deuterated compound thereof, or a pharmaceutically acceptable salt thereof. In some embodiments, the FAK inhibitor is IN10018, Defactinib, AMP945, deuterated compound 1 of Defactinib (CAS No.: 2384121-03-1), deuterated compound 2 of Defactinib (CAS No.: 2384120-99-2), or a pharmaceutically acceptable salt thereof. For example, a pharmaceutically acceptable salt of IN10018 may be a tartrate, a pharmaceutically acceptable salt of AMP945 may be a tartrate, and a pharmaceutically acceptable salt of Defactinib may be a hydrochloride.

The term "Pan-RAS inhibitor" as used herein refers to an inhibitor that targets all RAS mutations. In some embodiments, the Pan-RAS inhibitor is RMC6236 (CAS No.: 2765081-21-6), Pan-RAS-IN-1 (CAS No.: 1835283-94-7), or ADT-007 (CAS No.: 1945941-09-2).

As used herein, "pharmaceutical combination" or "pharmaceutical combination product" can refer to a fixed combination in the form of one dosage unit (for example, all active pharmaceutical ingredients are present in one dosage form) or a product as a kit-of-parts for combined administration, or it may refer to a combination of one drug and instructions indicating that the drug can be used in combination with one or more other drugs.

As used herein, "combination therapy" or "combination medication" refers to the use of a drug in combination with one or more other drugs to treat a disease, including the combination of a drug with one or more other drugs and the combination of a drug with instructions indicating that the drug can be used in combination with one or more other drugs.

"Simultaneously or sequentially administration" in the present application refers to the simultaneous or sequential administration at certain time intervals of more than two drugs within one administration cycle (e.g., within 4 weeks, within 3 weeks, within 2 weeks, within 1 week, or within 24 hours). The modes of drug administration (e.g., oral, intravenous, intramuscular, or subcutaneous administration, etc.) may be the same or different, and the dosing frequency/cycle of more than two drugs may be the same or different. When the treatment method, product, or use of the present disclosure involves two drugs, the two drugs may be administered simultaneously or separately at certain time intervals.

As used herein, the term "treat", "treating", or "treatment" refers to the administration of one or more pharmaceutical substances to a subject suffering from a disease or having symptoms of the disease to cure, relief, alleviate, alter, treat, improve, ameliorate or affect the disease or symptoms of the disease. In some embodiments, the disease is a tumor or cancer.

As used herein, the term "tumor" refers to an abnormal lesion formed by the loss of normal regulation of local tissue cells' growth at the genetic level under the influence of various tumorigenic factors, resulting in clonal abnormal proliferation. The tumor includes, but is not limited to: bladder cancer, breast cancer, cervical cancer, colon cancer (including colorectal cancer), esophageal cancer, esophageal squamous cell carcinoma, head and neck cancer, liver cancer, lung cancer (including small cell lung cancer and non-small cell lung cancer), melanoma, myeloma, rhabdomyosarcoma, inflammatory myofibroblastoma, neuroblastoma, pancreatic cancer, prostate cancer, kidney cancer, renal cell carcinoma, fibrosarcoma (including osteosarcoma), skin cancer (including squamous cell carcinoma), gastric cancer, testicular cancer, thyroid cancer, uterine cancer, mesothelioma, bile duct carcinoma, leiomyosarcoma, liposarcoma, nasopharyngeal carcinoma, neuroendocrine carcinoma, ovarian cancer, salivary gland cancer, metastatic tumors caused by spindle cell carcinoma, anaplastic large cell lymphoma, undifferentiated thyroid carcinoma, non-Hodgkin's lymphoma, Hodgkin's lymphoma, glioma, and hematologic malignancies such as acute myeloid leukemia (AML), acute lymphoblastic leukemia (ALL), diffuse large B-cell lymphoma (DLBCL), follicular lymphoma (FL), chronic lymphocytic leukemia (CLL), and chronic myeloid leukemia (CML); alternatively, the tumor is fibrosarcoma, lung cancer, breast cancer, glioma, esophageal cancer, pancreatic cancer, head and neck cancer, ovarian cancer, or colon cancer (including colorectal cancer); more alternatively, the tumor is colon cancer (including colorectal cancer), lung cancer, pancreatic cancer, or fibrosarcoma; more alternatively, the tumor is colon cancer (including colorectal cancer) or fibrosarcoma.

As used herein, the term "patient" or "subject" refers to both mammals and non-mammals. Mammals refer to any member of the mammal family, including but not limited to human; non-human primate such as chimpanzee and other apes and monkey species; farm animals such as cattle, horse, sheep, goat, and pig; livestock such as rabbit, dog, and cat; laboratory animal, including rodent such as rat, mice, and guinea pig; and the like. Examples of non-mammals include, but are not limited to, birds. The term "subject" does not limit the subject to a particular age or sex. In some embodiments, the subject is a human.

As used herein, the term "pharmaceutically acceptable" refers to a substance that is non-toxic, biologically tolerable, and suitable for administration to a subject.

As used herein, the term "pharmaceutically acceptable salt" refers to an acid addition salt that is non-toxic, biologically tolerable, and suitable for administration to a subject, including but not limited to: an acid addition salt formed with an inorganic acid, such as hydrochloride, hydrobromide, carbonate, bicarbonate, phosphate, sulfate, sulfite, nitrate, and the like; and an acid addition salt formed with an organic acid, such as formate, acetate, malate, maleate, fumarate, tartrate, succinate, citrate, lactate, methanesulfonate, p-toluenesulfonate, 2-hydroxyethanesulfonate, benzoate, salicylate, stearate, and a salt formed with alkane dicarboxylic acid of the formula HOOC-(CH₂)ₙ-COOH (wherein n is 0-4), and the like.

Furthermore, the pharmaceutically acceptable acid addition salts can be obtained by dissolving the free base in a suitable solvent and treating the solution with an acid according to conventional procedures for the preparation of the acid addition salts from the basic compound. One skilled in the art can determine various synthetic methods that can be used to prepare non-toxic pharmaceutically acceptable acid addition salts without undue experimentation. In some embodiments, the pharmaceutically acceptable salt of IN10018 is a tartrate. In some embodiments, the pharmaceutically acceptable salt of AMP945 is a tartrate. In some embodiments, the pharmaceutically acceptable salt of Defactinib is a hydrochloride.

As used herein, the term "pharmaceutically acceptable" refers to it must be chemically and/or toxicologically compatible with other components in the formulation, and/or with the subject receiving treatment thereof. The term "therapeutically effective amount" as used herein refers to an amount generally sufficient to produce a beneficial therapeutic effect on the subject. The therapeutically effective amount of the present disclosure can be determined by conventional methods (e.g., modeling, dose-escalation studies, or clinical trials) in conjunction with conventional influence factors (e.g., mode of administration, pharmacokinetics of the compound, severity and course of disease, medical history of the subject, health status of the subject, response of the subject to the drug, etc.).

As used herein, the term "inhibition" refers to a decrease in baseline activity of a biological activity or process.

As used herein, the term "kit" refers to a box for containing chemical reagents for detecting chemical components, drug residues, viral species, and the like. The kit described in the present disclosure may include (i) a FAK inhibitor and/or a Pan-RAS inhibitor; and (ii) instructions indicating that the FAK inhibitor and Pan-RAS inhibitor can be used to treat a tumor in a subject. In one embodiment, the kit includes (i) a FAK inhibitor; and (ii) instructions indicating that the FAK inhibitor and Pan-RAS inhibitor can be used to treat a tumor in a subject. In one embodiment, the kit includes (i) a Pan-RAS inhibitor; and (ii) instructions indicating that the Pan-RAS inhibitor and FAK inhibitor can be used to treat a tumor in a subject. In one embodiment, the kit includes (i) a FAK inhibitor and a Pan-RAS inhibitor; and (ii) instructions indicating that the FAK inhibitor and Pan-RAS inhibitor can be used to treat a tumor in a subject.

The compounds in the kit may be contained in separate containers. Optionally, two or more compounds are included in the same container. For example, the kit may include a first container, a second container, and a packaging insert, wherein the first container contains at least one dose of a FAK inhibitor, the second container contains at least one dose of a Pan-RAS inhibitor, and the packaging insert contains instructions for treating the tumor of the patient with the drug. The first and second containers may have the same or different shapes (e.g., vials, syringes, and bottles) and/or materials (e.g., plastic or glass). The kit may also include other materials that may facilitate drug administration, such as diluents, filters, IV bags and lines, needles, and syringes.

The exact amount of FAK inhibitor and Pan-RAS inhibitor administered to a subject will depend on various factors, such as the given drug or compound, the drug preparation, the route of administration, the type of disease, the condition, the identity of the subject or host being treated, etc., but can nevertheless be routinely determined by one skilled in the art. For example, the determination of an effective amount will also depend on the extent, severity, and type of cell proliferation. A skilled artisan will be able to determine an appropriate dosage based on these and other factors.

The FAK inhibitor and the Pan-RAS inhibitor may be administered in a suitable manner such as oral, intravenous, intramuscular, or subcutaneous administration.

For example, when administered orally, the drug may be administered orally with a pharmaceutically acceptable carrier such as an inert diluent or an absorbable edible carrier. They may be encapsulated in hard or soft-shell gelatin capsules, compressed into tablets, or mixed directly with the patient's food. For example, the drug can be combined with one or more excipients and used in the form of ingestible tablets, oral tablets, lozenges, capsules, elixirs, suspensions, syrups, or rice paper capsules. The tablets, lozenges, pills, capsules, etc., may further include: binders such as tragacanth, Arabic gum, corn starch, or gelatin; excipients such as dicalcium phosphate; disintegrants such as corn starch, potato starch, alginate, etc.; lubricants such as magnesium stearate; or sweeteners such as sucrose, fructose, lactose, or aspartame; or flavoring agents.

For example, when administering intravenously or intraperitoneally by infusion or injection, the solution of the drug can be prepared in water, optionally mixed with a non-toxic surfactant.

Exemplary drug dosage forms for injection or infusion include: sterile aqueous solutions, dispersions, or sterile powders containing an active ingredient suitable for the temporary preparation of sterile injectable or infusion solutions or dispersions. In any case, the final dosage form should be sterile, flowable, and stable under both manufacturing and storage conditions.

Sterile injectable solutions can be prepared by incorporating the required amount of the drug with the various other desired components described above into a suitable solvent, followed by filtration and sterilization. For sterile powders used to prepare sterile injectable solutions, preferred preparation methods may be vacuum drying and freeze-drying techniques, which can produce a powder containing the active ingredient plus any other desired components present after previous sterile filtration.

The amount of FAK inhibitor and PAN-RAS inhibitor required for treatment can vary not only with the particular reagents selected, but also with the route of administration, the nature of the disease being treated, and the age and condition of the patient, and ultimately can be determined by the attending physician or clinician. In general, however, the dosage may be in the range of about 0.1 to about 50 mg/kg body weight per day.

The technical and scientific terms not specifically defined as used herein have the meanings commonly understood by those skilled in the art to which the disclosure pertains.

### EXAMPLE

The following examples are provided to further describe the present disclosure. It should also be understood that these examples are only for the purpose of illustrating the present disclosure, rather than limiting the scope thereof.

The experimental methods without specific conditions in the following examples can be carried out according to the conventional conditions of this type of reaction or the conditions suggested by the manufacturer.

The experimental materials and reagents used in the following examples can be commercially available unless otherwise specified.

The abbreviations used in the examples have the following meanings:

| | |
|---|---|
| FBS | Fetal bovine serum |
| DPBS | Duchenne phosphate buffer solution |
| mm | Millimeter |
| mL | Milliliter |
| µL | Microliter |
| mg | Milligram |
| kg | Kilogram |
| °C | Celsius |
| IC50 | Half-maximal inhibitory concentration |

### Example 1: Study on the in vitro inhibitory activity of RMC6236 in combination with FAK inhibitor on the proliferation of the HT1080 fibrosarcoma cells

### Experimental Design:

The grouping is shown in Table 1.

**Table 1: Grouping scheme for HT1080 cell proliferation inhibition assay**

| **Number** | **Test drug** |
|---|---|
| 1 | RMC6236 |
| 2 | RMC6236+1/4*IC50 IN10018 |
| 3 | RMC6236+1/2*IC50 IN10018 |
| 4 | RMC6236+1/4*IC50 AMP945 |
| 5 | RMC6236+1/2*IC50 AMP945 |
| 6 | RMC6236+1/4*IC50 Defactinib |
| 7 | RMC6236+1/2*IC50 Defactinib |

The test sample information is shown in Table 2.

**Table 2:**

| | | | | |
|---|---|---|---|---|
| **Name** | IN10018 | RMC6236 | AMP945 | Defactinib |
| **Code name** | IN10018 | RMC6236 | AMP945 | Defactinib |
| **Source** | Synthesized according to the method in WO2010058032 | MCE | MCE | Shanghai Chaolan Chemical Co., LTD |
| **Batch No./Item No.** | | Cat: HY-148439 | Cat: HY-145652 | Lot: 202009 |
| | | Lot:285814 | Lot:143253 | |
| **Properties** | White powder | White powder | White powder | White powder |
| **Vehicle** | DPBS | DMSO | DMSO | DMSO |
| **Storage conditions** | Normal temperature | -20°C | -20°C | -20°C |

### Experimental methods and steps:

HT1080 cells were thawed and maintained for passage. The cells were cultured in an *in vitro* monolayer adherent culture under culture conditions of 10% fetal bovine serum in RPMI 1640 medium, at 37 °C and 5% CO₂. The cells were digested with trypsin and passaged two to three times per week. When the cell growth was in an exponential growth phase and the cell confluence reached 80%-90%, the cells were digested and plated.

HT1080 cells were counted after being digested with trypsin. Based on the count results, the cells were diluted to 30,000 cells per milliliter using RPMI1640 + 10%FBS. The cells were then plated in a 96-well cell flat-bottomed culture plate, with 0.1 ml of cell suspension (i.e., 3,000 cells) added to each well. After the cell plating was completed, the culture plates were placed in a 37°C, 5% CO₂ incubator for further culture.

24 hours after cell plating, the test compounds were added to different wells of the cell plate. The test compound was uniformly prepared and dispensed before the experiment. In short, the drug was dissolved to 10 mM using the corresponding solvent, dispensed in 50 µL aliquots, and stored at -20 °C in the dark. In this experiment, one aliquot was used to add the drug to the cells and used for treatment.

After the drug addition was completed, the drugs were gently mixed until uniform, and then the cells were placed in an incubator at 37 °C and 5% CO₂ for cultivation.

After 120 hours of drug treatment, 10 µL of CCK-8 assay reagent was added to each well of the cell plate using a multi-channel pipette. The cell plate was then placed in a 5% CO₂ incubator at 37 °C for further incubation for 4 hours. Finally, the absorbance of each well at 450 nm was measured using a microplate reader.

### Data Analysis:

After the experiment was completed, GraphPad Prism 8 software was used to analyze the percent inhibition of cells by the test drug.

The percent inhibition was calculated as follows: 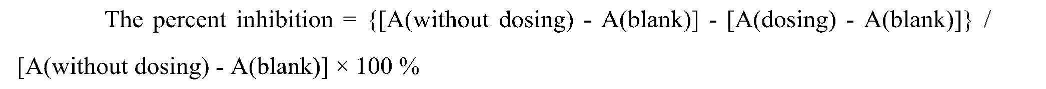
The percent inhibition = {[A(without dosing) - A(blank)] - [A(dosing) - A(blank)]} / [A(without dosing) - A(blank)] × 100 %

A (dosing): Absorbance values of wells containing cells, CCK-8 solution, and drug solution.

A (blank): Absorbance value of wells containing culture medium and CCK-8 solution, but without cells.

A (without dosing): Absorbance value of wells containing cells and CCK-8 solution, but without drug solution.

This experiment evaluated the inhibitory effects of RMC6236 alone and in combination with different FAK inhibitors on the proliferation of HT1080 cells under in vitro conditions.

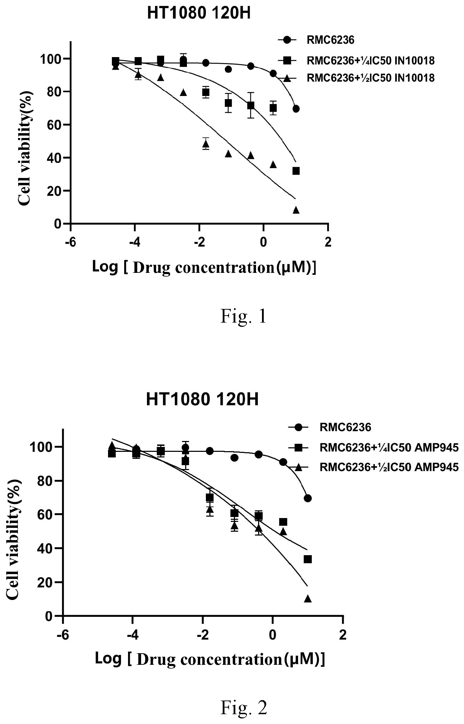

The relevant detection results of each group of cells 120 hours after drug treatment are shown in Figs. 1 to 3 and Tables 3 to 5.

**Table 3: Percentage (%) of HT 1080 cell viability (RMC6236 + different concentrations of IN10018)**

| Drug concentration (µM) | RMC6236 | | RMC6236+¼IC50 IN10018 | | RMC6236+½IC50 IN10018 | |
|---|---|---|---|---|---|---|
| 10 | 69 | 70 | 32 | 32 | 9 | 8 |
| 2 | 92 | 90 | 73 | 67 | 35 | 37 |
| 0.4 | 95 | 96 | 77 | 66 | 42 | 41 |
| 0.08 | 93 | 94 | 77 | 69 | 41 | 44 |
| 0.016 | 97 | 98 | 82 | 77 | 46 | 51 |
| 0.0032 | 97 | 102 | 99 | 96 | 80 | 79 |
| 0.00064 | 98 | 97 | 101 | 98 | 87 | 90 |
| 0.000128 | 98 | 99 | 100 | 97 | 88 | 93 |
| 2.56E-05 | 96 | 98 | 100 | 97 | 95 | 96 |

**Table 4: Percentage (%) of HT 1080 cell viability (RMC6236 + different concentrations of AMP945)**

| Drug concentration (µM) | RMC6236 | | RMC6236+¼IC50 AMP945 | | RMC6236+½IC50 AMP945 | |
|---|---|---|---|---|---|---|
| 10 | 69 | 70 | 33 | 34 | 11 | 10 |
| 2 | 92 | 90 | 55 | 56 | 50 | 50 |
| 0.4 | 95 | 96 | 61 | 57 | 49 | 55 |
| 0.08 | 93 | 94 | 64 | 57 | 51 | 56 |
| 0.016 | 97 | 98 | 74 | 66 | 60 | 66 |
| 0.0032 | 97 | 102 | 95 | 88 | 91 | 96 |
| 0.00064 | 98 | 97 | 100 | 95 | 96 | 100 |
| 0.000128 | 98 | 99 | 98 | 94 | 98 | 101 |
| 2.56E-05 | 96 | 98 | 95 | 97 | 100 | 102 |

**Table 5: Percentage (%) of HT 1080 cell viability (RMC6236 + different concentrations of Defactinib)**

| Drug concentration (µM) | RMC6236 | | RMC6236+¼IC50 Defactinib | | RMC6236+½IC50 Defactinib | |
|---|---|---|---|---|---|---|
| 10 | 69 | 70 | 73 | 70 | 38 | 43 |
| 2 | 92 | 90 | 86 | 86 | 53 | 61 |
| 0.4 | 95 | 96 | 89 | 88 | 48 | 58 |
| 0.08 | 93 | 94 | 86 | 85 | 49 | 59 |
| 0.016 | 97 | 98 | 88 | 86 | 58 | 66 |
| 0.0032 | 97 | 102 | 95 | 97 | 83 | 97 |
| 0.00064 | 98 | 97 | 97 | 98 | 87 | 102 |
| 0.000128 | 98 | 99 | 96 | 95 | 88 | 102 |
| 2.56E-05 | 96 | 98 | 97 | 98 | 91 | 101 |

The effect of different concentrations of RMC6236 alone and in combination with different FAK inhibitors on cell proliferation were investigated. The combination therapy group showed better cell-killing effect than the single-drug group, and cell viability decreased with increasing drug concentration. Therefore, the combination therapy group had a stronger in vitro inhibitory effect on cancer cell growth.

### Example 2: Study on the in vitro inhibitory activity of RMC6236 in combination with FAK inhibitors on the proliferation of CT26 colon cancer cells.

### Experimental Design:

The grouping is shown in Table 6.

**Table 6: Grouping scheme for CT26 cell proliferation inhibition assay**

| **Number** | **Test drug** |
|---|---|
| 1 | RMC6236 |
| 2 | RMC6236+1/4*IC50 IN10018 |
| 3 | RMC6236+1/2*IC50 IN10018 |
| 4 | RMC6236+1/4*IC50 AMP945 |
| 5 | RMC6236+1/2*IC50 AMP945 |
| 6 | RMC6236+1/4*IC50 Defactinib |
| 7 | RMC6236+1/2*IC50 Defactinib |

The test sample information is shown in Table 2.

### Experimental methods and steps:

CT26 cells were thawed and maintained for passage. The cells were cultured in an *in vitro* monolayer adherent culture under culture conditions of 10% fetal bovine serum in RPMI 1640 medium, at 37 °C and 5% CO₂. The cells were digested with trypsin and passaged two to three times per week. When the cell growth was in an exponential growth phase and the cell confluence reached 80%-90%, the cells were digested and plated.

CT26 cells were counted after being digested with trypsin. Based on the count results, the cells were diluted to 30,000 cells per milliliter using RPMI1640 + 10%FBS. The cells were then plated in a 96-well cell flat-bottomed culture plate, with 0.1 ml of cell suspension (i.e., 3,000 cells) added to each well. After the cell plating was completed, the culture plates were placed in a 37°C, 5% CO₂ incubator for further culture.

24 hours after cell plating, the test compounds were added to different wells of the cell plate. The test compound was uniformly prepared and dispensed before the experiment. In short, the drug was dissolved to 10 mM using the corresponding solvent, dispensed in 50 µL aliquots, and stored at -20 °C in the dark. In this experiment, one aliquot was used to add the drug to the cells and used for treatment.

After the drug addition was completed, the drugs were gently mixed until uniform, and then the cells were placed in an incubator at 37 °C and 5% CO₂ for cultivation.

After 120 hours of drug treatment, 10 µL of CCK-8 assay reagent was added to each well of the cell plate using a multi-channel pipette. The cell plate was then placed in a 5% CO2 incubator at 37 °C for further incubation for 4 hours. Finally, the absorbance of each well at 450 nm was measured using a microplate reader.

### Data Analysis:

After the experiment was completed, GraphPad Prism 8 software was used to analyze the percent inhibition of cells by the test drug.

The percent inhibition was calculated as follows: 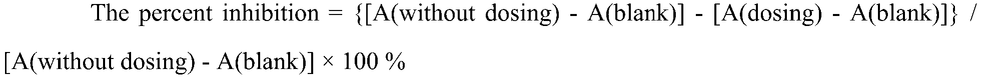 The percent inhibition = {[A(without dosing) - A(blank)] - [A(dosing) - A(blank)]} / [A(without dosing) - A(blank)] × 100 %

A (dosing): Absorbance values of wells containing cells, CCK-8 solution, and drug solution.

A (blank): Absorbance value of wells containing culture medium and CCK-8 solution, but without cells.

A (without dosing): Absorbance value of wells containing cells and CCK-8 solution, but without drug solution.

This experiment evaluated the inhibitory effects of RMC6236 alone and in combination with different FAK inhibitors on the proliferation of CT26 cells under in vitro conditions.

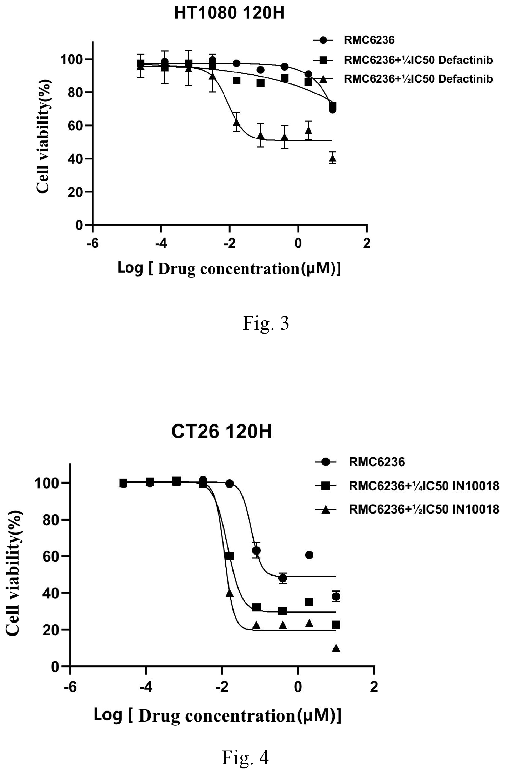

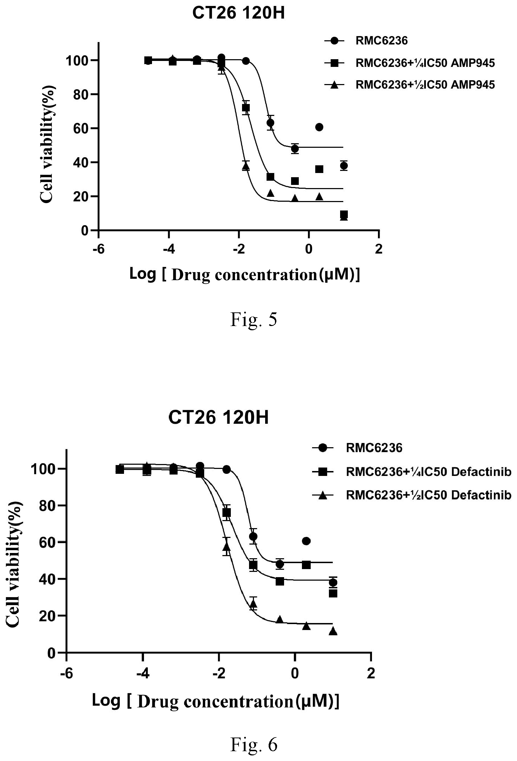

The relevant detection results of each group of cells 120 hours after drug treatment are shown in Figs. 4 to 6 and Tables 7 to 9.

**Table 7: Percentage (%) of CT26 cell viability (RMC6236 + different concentrations of IN10018)**

| Drug concentration (µM) | RMC6236 | RMC6236+¼IC50 IN10018 | | RMC6236+½IC50 IN10018 | | |
|---|---|---|---|---|---|---|
| 10 | 36 | 40 | 23 | 22 | 10 | 10 |
| 2 | 59 | 62 | 36 | 34 | 24 | 23 |
| 0.4 | 46 | 50 | 31 | 29 | 23 | 22 |
| 0.08 | 60 | 66 | 32 | 32 | 22 | 23 |
| 0.016 | 100 | 99 | 60 | 60 | 40 | 40 |
| 0.0032 | 101 | 102 | 99 | 100 | 100 | 100 |
| 0.00064 | 100 | 101 | 101 | 101 | 101 | 100 |
| 0.000128 | 99 | 101 | 100 | 101 | 101 | 100 |
| 2.56E-05 | 100 | 99 | 100 | 100 | 100 | 100 |

**Table 8: Percentage (%) of CT26 cell viability (RMC6236 + different concentrations of AMP945)**

| Drug concentration (µM) | RMC6236 | | RMC6236+¼IC50 AMP945 | | RMC6236+½IC50 AMP945 | |
|---|---|---|---|---|---|---|
| 10 | 36 | 40 | 9 | 10 | 8 | 8 |
| 2 | 59 | 62 | 37 | 35 | 20 | 20 |
| 0.4 | 46 | 50 | 29 | 29 | 20 | 18 |
| 0.08 | 60 | 66 | 32 | 31 | 22 | 22 |
| 0.016 | 100 | 99 | 75 | 69 | 36 | 40 |
| 0.0032 | 101 | 102 | 97 | 99 | 93 | 99 |
| 0.00064 | 100 | 101 | 99 | 100 | 100 | 101 |
| 0.000128 | 99 | 101 | 99 | 99 | 101 | 101 |
| 2.56E-05 | 100 | 99 | 100 | 100 | 100 | 100 |

**Table 9: Percentage (%) of CT26 cell viability (RMC6236 + different concentrations of Defactinib)**

| Drug concentration (µM) | RMC6236 | | RMC6236+¼IC50 Defactinib | | RMC6236+½IC50 Defactinib | |
|---|---|---|---|---|---|---|
| 10 | 36 | 40 | 33 | 31 | 12 | 12 |
| 2 | 59 | 62 | 49 | 46 | 15 | 14 |
| 0.4 | 46 | 50 | 38 | 39 | 18 | 18 |
| 0.08 | 60 | 66 | 50 | 45 | 24 | 29 |
| 0.016 | 100 | 99 | 79 | 73 | 54 | 61 |
| 0.0032 | 101 | 102 | 96 | 99 | 101 | 101 |
| 0.00064 | 100 | 101 | 98 | 100 | 101 | 101 |
| 0.000128 | 99 | 101 | 97 | 101 | 102 | 101 |
| 2.56E-05 | 100 | 99 | 99 | 100 | 101 | 100 |

The effect of different concentrations of RMC6236 alone and in combination with different FAK inhibitors on cell proliferation were investigated. The combination therapy group showed better cell-killing effect than the single-drug group, and cell viability of the combination therapy group decreased with increasing drug concentration. Therefore, the combination therapy group has a stronger in vitro inhibitory effect on tumor cell growth.

### Example 3: Study on the in vitro inhibitory activity of RMC6236 in combination with FAK siRNA on the proliferation of CT26 colon cancer cells.

### Experimental Design:

The grouping is shown in Table 10.

**Table 10: Grouping scheme for CT26 cell proliferation inhibition assay**

| **Number** | **Test drug** |
|---|---|
| 1 | RMC6236 |
| 2 | RMC6236+control siRNA(100nM) |
| 3 | RMC6236+FAK siRNA(100nM) |

The test sample information is shown in Table 11.

**Table 11:**

| | | |
|---|---|---|
| **Name** | RMC6236 | Control siRNA/FAK siRNA |
| **Code name** | RMC6236 | Control siRNA/FAK siRNA |
| **Source** | MCE | GenePharma Co., Ltd. |
| **Batch No./Item No.** | Cat: HY-148439 Lot:285814 | NC A06001/Lot:33545 |
| **Properties** | White powder | Dry powder |
| **Vehicle** | DMSO | DEPC water |
| **Storage conditions** | -20°C | -20°C |

Reagent information is shown in Table 12.

**Table 12:**

| **Reagent Name** | **Manufacturer** | **Item number** | **batch number** |
|---|---|---|---|
| Lipofectamine 3000 | Thermo | 100022050 | 2533503 |
| Opti-MEM | Gibco | 31985-070 | 2192943 |
| FAK siRNA | sense(5'-3') | GUCCAACUAUGAAGUAUUATT | |
| | antisense(5'-3') | UAAUACUUCAUAGUUGGACTT | |
| Control siRNA | sense(5'-3') | UUCUCCGAACGUGUCACGUTT | |
| | antisense(5'-3') | ACGUGACACGUUCGGAGAATT | |

### Experimental methods and steps:

CT26 cells were thawed and maintained for passage. The cells were cultured in an *in vitro* monolayer adherent culture under culture conditions of 10% fetal bovine serum in RPMI 1640 medium, at 37 °C and 5% CO₂. The cells were digested with trypsin and passaged two to three times per week. When the cell growth was in an exponential growth phase and the cell confluence reached 80%-90%, the cells were digested and plated.

CT26 cells were counted after being digested with trypsin. Based on the count results, the cells were diluted to 50,000 cells per milliliter using RPMI1640 + 10%FBS, and then 0.1 ml of cell suspension (i.e., 5,000 cells) was added to each well. After the cell plating was completed, the culture plates were placed in a 37°C, 5% CO₂ incubator for further culture.

CT26 cells were transfected with siRNA using Lipofectamine 3000 reagent. Solution A was prepared by diluting the siRNA to 100 nM with 125 µL Opti-MEM; Solution B was prepared by adding 6 µL Lipofectamine 3000 to 125 µL Opti-MEM and mixing well. Solutions A and B were gently mixed separately to prepare the transfection complex, standing for 15 minutes at room temperature. 10 µL of the transfection complex was then added to each well of a 96-well plate that has been plated with cells.

24 hours after cell plating, the test compounds were added to different wells of the cell plate. The test compound was uniformly prepared and dispensed before the experiment. In short, the drug was dissolved to 10 mM using the corresponding solvent, dispensed in 50 µL aliquots, and stored at -20 °C in the dark. In this experiment, one aliquot was used to add the drug to the cells and used for treatment. After the drugs were added, they were gently mixed, and then the cells were incubated in a 37 °C, 5% CO₂ incubator.

After 120 hours of drug treatment, 10 µL of CCK-8 assay reagent was added to each well of the cell plate using a multi-channel pipette. The cell plate was then placed in a 5% CO2 incubator at 37 °C for further incubation for 4 hours. Finally, the absorbance of each well at 450 nm was measured using a microplate reader.

### Data Analysis:

After the experiment was completed, GraphPad Prism 8 software was used to analyze the percent inhibition of cells by the test drug.

The percent inhibition was calculated as follows: 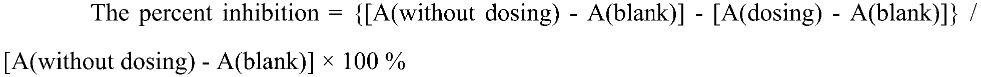 The percent inhibition = {[A(without dosing) - A(blank)] - [A(dosing) - A(blank)]} / [A(without dosing) - A(blank)] × 100 %

A (dosing): Absorbance values of wells containing cells, CCK-8 solution, and drug solution.

A (blank): Absorbance value of wells containing culture medium and CCK-8 solution, but without cells.

A (without dosing): Absorbance value of wells containing cells and CCK-8 solution, but without drug solution.

This experiment evaluated the killing effect of RMC6236 alone on CT26 cells after the FAK target was silenced under in vitro conditions.

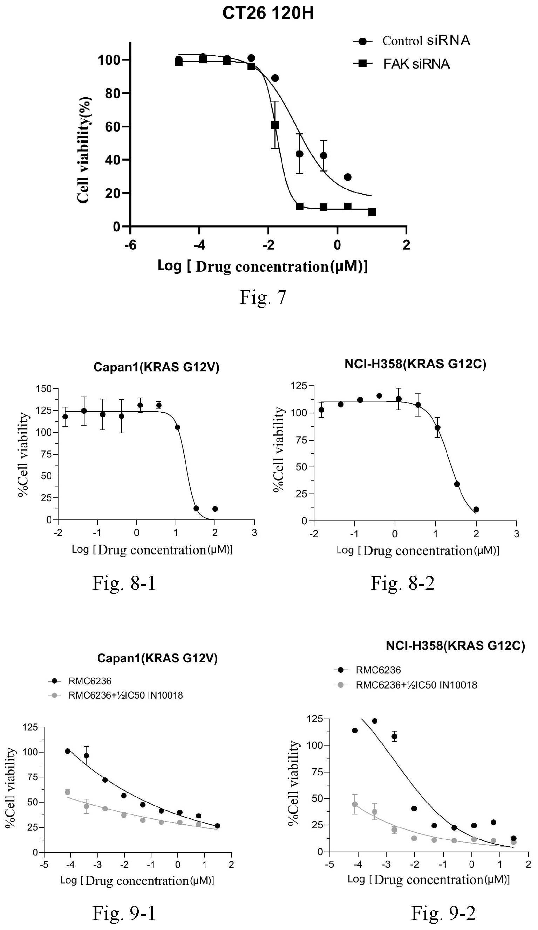

The cell killing results of CT26 cells after the FAK target was silenced, after treatment with the drug for 120 hours, are shown in Fig. 7 and Table 13.

**Table 13: Percentage (%) of CT26 cell viability**

| Drug concentration (µM) | Control siRNA (100 nM) | | FAK-siRNA (100nM) | |
|---|---|---|---|---|
| 10 | 8 | 9 | 9 | 8 |
| 2 | 30 | 29 | 12 | 12 |
| 0.4 | 49 | 36 | 12 | 11 |
| 0.08 | 52 | 35 | 11 | 13 |
| 0.016 | 88 | 90 | 71 | 51 |
| 0.0032 | 102 | 100 | 97 | 95 |
| 0.00064 | 101 | 100 | 98 | 100 |
| 0.000128 | 102 | 101 | 99 | 101 |
| 2.56E-05 | 100 | 100 | 98 | 99 |

In comparison with the control siRNA group, the drug sensitivity of CT26 to RMC6236 is significantly improved after FAK silencing. Therefore, RMC6236 has a stronger in vitro tumor cell killing effect under the condition of FAK target silencing.

### Example 4: Study on the in vitro inhibitory activity of different Pan-RAS inhibitors in combination with IN10018 on the proliferation of RAS mutant cell lines.

### Experimental Design:

The grouping is shown in Table 14.

**Table 14: Grouping scheme for HT1080 cell proliferation inhibition assay**

| **Number** | **Test drug** |
|---|---|
| 1 | IN10018 |
| 2 | RMC6236 |
| 3 | RMC6236+½IC50 IN10018 |
| 4 | Pan-RAS-IN-1 |
| 5 | Pan-RAS-IN-1+½IC50 IN10018 |
| 6 | ADT-007 |
| 7 | ADT-007+½IC50 IN10018 |

Information on test samples IN10018 and RMC6236 is shown in Table 2, and information on the remaining test samples is shown in Table 15.

**Table 15:**

| | | |
|---|---|---|
| **Name** | Pan-RAS-IN-1 | ADT-007 |
| **Code name** | Pan-RAS-IN-1 | ADT-007 |
| **Source** | MCE | MCE |
| **Batch No./Item No.** | 24626 | 452630 |
| **Properties** | White powder | White powder |
| **Vehicle** | DMSO | DMSO |
| **Storage conditions** | -20°C | -20°C |

### Experimental methods and procedures:

Capan1 cells (Nanjing Kebai Biotechnology Co., Ltd., Item No. CBP60543, human pancreatic cancer cells) and NCI-H358 cells (Nanjing Kebai Biotechnology Co., Ltd., Item No. CBP60136, human non-small cell lung cancer cells) were resuscitated and passaged. Cells were cultured in vitro in an adherent monolayer. Capan1 cells were cultured in DMEM medium supplemented with 10% fetal bovine serum, and NCI-H358 cells were cultured in RPMI1640 medium supplemented with 10% fetal bovine serum. Both cells were cultured at 37 °C and 5% CO₂. Cells were digested with trypsin and passaged two to three times per week. When cell growth was in the exponential growth phase, and the adherent confluence reaches 80%-90%, the cells were collected and plated.

Capan1 cells and NCI-H358 cells were digested with trypsin, and then the cells were collected and counted. Based on the count results, the cells were diluted to 30,000 cells per milliliter using culture medium. The cells were then plated in a 96-well cell flat-bottomed culture plate, with 0.1 ml of cell suspension (i.e., 3,000 cells) added to each well. After the cell plating was completed, the culture plates were placed in a 37°C, 5% CO₂ incubator for further culture.

24 hours after cell plating, the test compounds were added to different wells of the cell plate. The test compound was uniformly prepared and dispensed before the experiment. In short, the drug was dissolved to 10 mM using DMSO, dispensed in 50 µL aliquots, and stored at -20 °C in the dark. In this experiment, one aliquot was used to add the drug to the cells and used for treatment.

After the drug addition was completed, the drugs were gently mixed until uniform, and then the cells were placed in an incubator at 37 °C and 5% CO₂ for cultivation.

After 120 hours of drug treatment, 10 µL of CCK-8 assay reagent was added to each well of the cell plate using a multi-channel pipette. The cell plate was then placed in a 5% CO₂ incubator at 37 °C for further incubation for 4 hours. Finally, the absorbance of each well at 450 nm was measured using a microplate reader.

The data analysis is the same as in Example 1.

This experiment evaluated the in vitro inhibitory effects of three Pan-RAS inhibitors, RMC6236, Pan-RAS-IN-1, and ADT-007, as monotherapy and in combination with IN10018 respectively, on the proliferation of RAS mutant cell lines under in vitro conditions.

The relevant detection results of each group of cells 120 hours after drug treatment are shown in Figs. 8 to 11 and Tables 16 to 19.

**Table 16: Percentage (%) of cell viability of IN10018 monotherapy in different cell**

| Drug concentration (µM) | Capan1 | | NCI-H358 | |
|---|---|---|---|---|
| | IN10018 | | IN10018 | |
| 100 | 12 | 13 | 11 | 10 |
| 33.33333 | 12 | 14 | 33 | 35 |
| 11.11111 | 105 | 107 | 93 | 80 |
| 3.703704 | 128 | 134 | 115 | 100 |
| 1.234568 | 125 | 137 | 120 | 106 |
| 0.411523 | 105 | 132 | 116 | 116 |
| 0.137174 | 108 | 133 | 111 | 113 |
| 0.045725 | 113 | 136 | / | 108 |
| 0.015242 | 110 | 126 | 98 | 108 |

**Table 17: Percentage (%) of Cell Viability**

| Drug concentration (µM) | Capan1 | | | | NCI-H358 | | | |
|---|---|---|---|---|---|---|---|---|
| | RMC6236 | | RMC6236 + ½IC50 IN10018 | | RMC6236 | | RMC6236 + ½IC50 IN10018 | |
| 30 | 27 | 26 | 27 | 26 | 12 | 13 | 9 | 9 |
| 6 | 37 | 36 | 29 | 27 | 27 | 28 | 11 | 10 |
| 1.2 | 39 | 41 | 31 | 29 | 24 | 25 | 12 | 11 |
| 0.24 | 41 | 42 | 31 | 29 | 22 | 23 | 11 | 10 |
| 0.048 | 47 | 48 | 33 | 31 | 24 | 25 | 12 | 10 |
| 0.0096 | 55 | 58 | 39 | 35 | 40 | 41 | 13 | 12 |
| 0.00192 | 71 | 73 | 45 | 42 | 105 | 112 | 23 | 18 |
| 0.000384 | 90 | 103 | 51 | 41 | 123 | / | 43 | 32 |
| 0.0000768 | 101 | 101 | 62 | 58 | 114 | / | 51 | 38 |

**Table 18: Percentage (%) of Cell Viability**

| Drug concentration (µM) | Capan1 | | | | NCI-H358 | | | |
|---|---|---|---|---|---|---|---|---|
| | Pan-RAS-IN-1 | | Pan-RAS-IN-1 + ½IC50 IN10018 | | Pan-RAS-IN-1 | | Pan-RAS-IN-1 + ½IC50 IN10018 | |
| 30 | 12 | 12 | 14 | 14 | 7 | 7 | 8 | 8 |
| 6 | 11 | 11 | 13 | 12 | 6 | 6 | 7 | 6 |
| 1.2 | 93 | 94 | 34 | 49 | 93 | 95 | 65 | 61 |
| 0.24 | 100 | 110 | 52 | 43 | 102 | 100 | 78 | 67 |
| 0.048 | 106 | 112 | 66 | 58 | 101 | 101 | 78 | 69 |
| 0.0096 | 106 | 114 | 69 | 62 | 102 | 102 | 81 | 70 |
| 0.00192 | 111 | 114 | 71 | 61 | 101 | 105 | 75 | 71 |
| 0.000384 | 100 | 97 | 71 | 67 | 101 | 107 | 80 | 70 |
| 0.0000768 | 99 | 99 | 83 | 76 | 101 | 106 | 82 | 73 |

**Table 19: Percentage (%) of Cell Viability**

| Drug concentration (µM) | Capan1 | | | | NCI-H358 | | | |
|---|---|---|---|---|---|---|---|---|
| | ADT-007 | | ADT-007 + ½IC50 IN10018 | | ADT-007 | | ADT-007 + ½IC50 IN10018 | |
| 30 | 22 | 30 | 14 | 15 | 25 | 23 | 21 | 20 |
| 6 | 34 | 38 | 26 | 29 | 38 | 37 | 20 | 20 |
| 1.2 | 42 | 58 | 32 | 34 | 33 | 32 | 19 | 18 |
| 0.24 | 60 | 76 | 38 | 37 | 28 | 32 | 19 | 20 |
| 0.048 | 87 | 103 | 72 | 60 | 30 | 31 | 20 | 20 |
| 0.0096 | 91 | 112 | 74 | 75 | 30 | 31 | 19 | 21 |
| 0.00192 | 98 | 103 | 77 | 74 | 28 | 30 | 18 | 20 |
| 0.000384 | 99 | 105 | 84 | 81 | 100 | 92 | 38 | 37 |
| 0.0000768 | 107 | 105 | 93 | 83 | 102 | 98 | 75 | 72 |

IN10018 monotherapy has an IC₅₀ of 18.38 µM for Capan1 cells and an IC₅₀ of 23.10 µM for NCI-H358 cells. The combination group of IN10018 and Pan-RAS inhibitor showed lower cell viability compared to the Pan-RAS inhibitor monotherapy group. All these results demonstrate that IN10018 effectively enhances the cell-killing effect of Pan-RAS inhibitors in RAS-mutant cell lines, thus the combination therapy group exhibits a stronger in vitro inhibitory effect on cancer cell growth.

### Example 5: Evaluation of the in vivo antitumor efficacy of the test article in CT26 colorectal cancer subcutaneous xenograft model.

CT26 cells (Nanjing Kebai Biotechnology Co., Ltd., Item No. CBP60043) were cultured with RPMI1640 medium containing 10% heat-inactivated fetal bovine serum in a 5% CO₂ incubator at 37°C. Cells in the exponential growth phase were harvested, resuspended in DPBS, and quantified using a cell counter before tumor inoculation.

3*10⁵ CT26 cells were inoculated subcutaneously on the right flank of each mouse (BALB/c mice, 6-7 weeks old, Shanghai Lingchang Biotechnology Co., Ltd.) in an amount of 0.1 mL DPBS. When the average tumor volume reached approximately 94mm³ (12 days post-inoculation), mice with moderate tumor volume were selected for enrollment, and treatment was initiated. The group and scheme information is shown in Table 20.

**Table 20:**

| **Gr oup** | **N¹** | **Test article** | **Dosage (mg/kg)** | **Dosing volume (mL/kg)** | **Dosing frequency** | **Route of administration** |
|---|---|---|---|---|---|---|
| 1 | 6 | Blank control group | N/A | 10 | PO | QD*12 days |
| 2 | 6 | RMC6236 | 10 | 10 | PO | QD*12 days |
| 3 | 6 | anti-mPD-1 | 10 | 10 | IP | BIW * 4 times |
| 4 | 6 | IN10018 | 25 | 10 | PO | QD*14 days |
| 5 | 6 | RMC6236+ IN10018 | 10+25 | 10 | PO + PO | QD*19 days + QD*19 days |
| 6 | 6 | RMC6236+ anti-mPD-1+ IN10018 | 10+10+25 | 10 | PO + IP + PO | QD*19 days + BIW * 6 times + QD * 19 days |

| | | | | | | |
|---|---|---|---|---|---|---|
| Note: 1. N: Number of animals in each group | | | | | | |

Information on test articles IN10018 and RMC6236 is shown in Table 2. Anti-mPD-1 (KYINNO Biotechnology, Lot: 20230919, concentration 5 mg/mL, solvent: DPBS, stored at 4 °C).

After the cell inoculation, the activity, ingestion and drinking conditions, weight gain or loss, eyes, fur and other abnormal conditions of the experimental animal were routinely observed. All clinical symptoms observed during the experiment were recorded in the raw data. The animal's weight and tumor size were measured every two days after cell inoculation. The tumor size was calculated using the formula: Tumor volume (mm³) = 0.5 × (tumor long diameter× tumor short diameter²).

Tumor volume inhibition rate TGI_{TV}(%): TGI% = (1-ΔT/ΔC)×100%; wherein ΔC is the tumor volume of the control group Cₜ-C₀ , C₀ is the average tumor volume of the control group at the time of grouping, Cₜ is the average tumor volume of the control group after treatment, and ΔT is the tumor volume of the treatment group Tₜ-T₀, T₀ is the average tumor volume of the control group at the time of grouping, and Tₜ is the average tumor volume of the control group after treatment.

Pharmacodynamic activity evaluation is performed according to tumor inhibition rate (TGI_{TV}%), and tolerance evaluation is performed according to animal weight change and death condition.

The tumor volume and animal body weight results were expressed as Mean ± SEM (mean standard error). All data were analyzed using GraphPad Prism 8.0. The differences in tumor volume among each group during the entire experiment were statistically analyzed through two-way ANOVA and Fisher's LSD, with p < 0.05 was considered statistically significant.

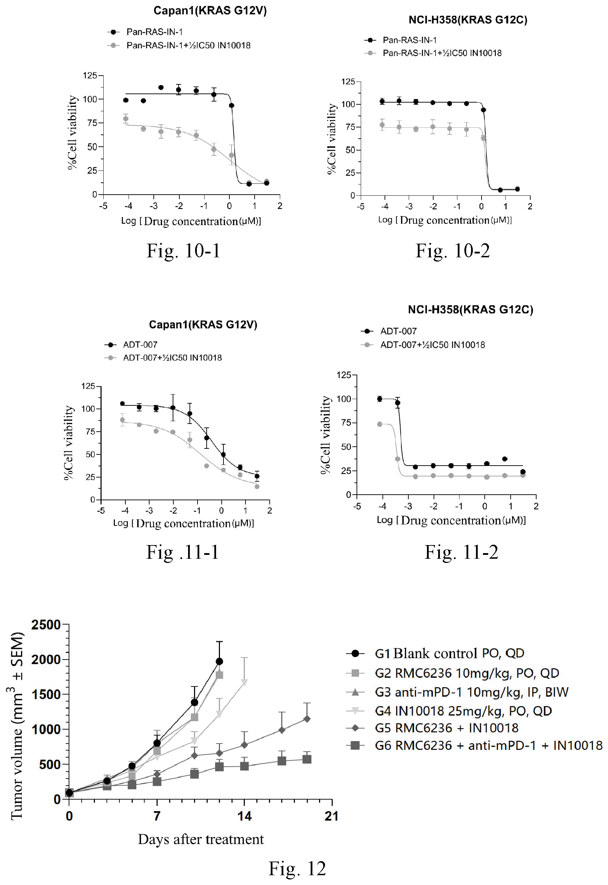

The tumor growth was observed every day. Due to excessive tumor size, mice in the blank control group, the RMC6236 10 mg/kg group, and the anti-mPD-1 10 mg/kg group were euthanized on day 12, while mice in the IN10018 25 mg/kg group were euthanized on day 14. Mice in other groups were observed until the end of the experiment (day 19). Tumor volume, TGI value, and statistical analysis for each group are shown in Table 21. Tumor volume for each group at different time points is shown in Fig. 12.

**Table 21:**

| **Group** | **Tumor volume (day 0) (mm³)¹** | **Tumor volume (day 12) (mm³)¹** | **TGIₜᵥ (%)²** | **P-value³** | **Tumor volume (day 19) (mm³)¹** | **P-value⁴** |
|---|---|---|---|---|---|---|
| Blank control group | 94.0±7.5 | 1970.7±289.5 | / | / | / | / |
| RMC6236 10 mg/kg | 94.2±6.9 | 1778.4±160.5 | 10.3 | 0.2995 | / | / |
| anti-mPD-1 10 mg/kg | 93.9±7.8 | 1809.3±438.6 | 8.6 | 0.3839 | / | / |
| IN10018 25 mg/kg | 94.1±9.8 | 1212.1±228.8 | 40.4 | <0.0001 | / | / |
| RMC6236+ IN10018 | 94.4±9.1 | 662.3±138.2 | 69.7 | <0.0001 | 1148.5±226.5 | 0.0064 |
| RMC6236+ anti-mPD-1+ IN10018 | 94.2±6.8 | 466.9±109.5 | 80.1 | <0.0001 | 574.0±111.4 | / |

| | | | | | | |
|---|---|---|---|---|---|---|
| Note: 1. Mean ± SEM; 2. Calculated based on data from day 12; 3. Calculated based on data from day 12, ****: p<0.0001, vs. blank control group, Two-way ANOVA; 4. Calculated based on data from day 19, **: p<0.01, ***: p<0.001, vs. RMC6236+ anti-mPD-1+ IN10018 10+10+25 mg/kg group, Two-way ANOVA. | | | | | | |

No abnormalities were found in the body weight of any of the experimental animals. The trend of body weight change in the treatment group was consistent with that in the vehicle control group, as detailed in Table 22. These results indicate that the animal is well tolerated for treatment.

**Table 22:**

| **Group** | **Survi val¹** | **Weight (Day 0) (g)²** | **Weight (Day 12) (g)²** | **Weight change rate (%)³** |
|---|---|---|---|---|
| Blank control group | 6/6 | 21.2±0.4 | 24.8±0.6 | 16.9 |
| RMC6236 10 mg/kg | 6/6 | 21.2±0.5 | 23.4±0.5 | 10.5 |
| anti-mPD-1 10 mg/kg | 6/6 | 21.9±0.1 | 24.6±0.7 | 12.5 |
| IN10018 25 mg/kg | 6/6 | 21.6±0.4 | 23.4±0.5 | 8.3 |
| RMC6236+ IN10018 | 6/6 | 21.7±0.5 | 21.3±0.5 | -1.8 |
| RMC6236+ anti-mPD-1+ IN10018 | 6/6 | 21.7±0.4 | 21.7±0.4 | 0.0 |

| | | | | |
|---|---|---|---|---|
| Note: 1. Number of animals surviving on day 12 / Number of animals surviving on day 0; 2. Mean ± SEM; 3. Weight change rate = (W₁₂-W₀) / W₀*100%. | | | | |

Compared with the blank control group, the IN10018 group, the RMC6236+IN10018 group, and the RMC6236+anti-mPD-1+IN10018 group showed significant differences, indicating that the above monotherapy and combination therapy have significant anti-tumor effects. Furthermore, the tumor volume in the RMC6236+anti-mPD-1+IN10018 three-drug group is consistently the smallest, showing a statistically significant difference compared to the RMC6236+IN10018 two-drug group. These results indicate that IN10018 synergistically treats cancer with RMC6236 and anti-mPD-1.

### Example 6: Evaluation of the in vivo anti-tumor efficacy of the test article in the Capan1 human pancreatic cancer subcutaneous xenograft model.

Capan1 cells (COBIOER, Cat No. CBP60543) were cultured with DMEM medium containing 10% heat-inactivated fetal bovine serum in a 5% CO₂ incubator at 37°C. Cells in the exponential growth phase were harvested, resuspended in DPBS:Matrigel = 1:1, and quantified using a cell counter before tumor inoculation.

10*10⁶ Capan1 cells were inoculated subcutaneously on the right flank of each mouse (BALB/c mouse, 6-7 weeks old, GemPharmatech Co., Ltd.) in an amount of 0.2 mL DPBS: Matrigel = 1: 1. When the average tumor volume reached approximately 260mm³ (12 days post-inoculation), mice with moderate tumor volume were selected for enrollment, and treatment was initiated. The group and scheme information is shown in Table 23.

**Table 23:**

| **Group** | **N¹** | **Test article** | **Dosage (mg/kg)** | **Dosing volume (mL/kg)** | **Dosing frequency** | **Route of administration** |
|---|---|---|---|---|---|---|
| 1 | 3 | Blank control group | N/A | 10 | IV | QW * 3 times |
| 2 | 2 | IN10018 | 25 | 10 | PO | QD * 20 days |
| 3 | 3 | RMC6236 | 10 | 10 | PO | QD * 20 days |
| 4 | 3 | RMC6236 + IN10018 | 3/6 + 25 | 10 | PO+PO | QD * 20 days+ QD * 20 days |

Information on test articles IN10018 and RMC6236 is shown in Table 2.

After the cell inoculation, the activity, ingestion and drinking conditions, weight gain or loss, eyes, fur and other abnormal conditions of the experimental animal were routinely observed. All clinical symptoms observed during the experiment were recorded in the raw data. The animal's weight and tumor size were measured every two days after cell inoculation. The tumor size was calculated using the formula: Tumor volume (mm³) = 0.5 × (tumor long diameter× tumor short diameter²).

Tumor volume inhibition rate TGI_{TV}(%): TGI% = (1-ΔT/ΔC)×100%; wherein ΔC is the tumor volume of the control group Cₜ-C₀ , C₀ is the average tumor volume of the control group at the time of grouping, Cₜ is the average tumor volume of the control group after treatment, and ΔT is the tumor volume of the treatment group Tₜ-T₀, T₀ is the average tumor volume of the control group at the time of grouping, and Tₜ is the average tumor volume of the control group after treatment.

Pharmacodynamic activity evaluation is performed according to tumor inhibition rate (TGI_{TV}%), and tolerance evaluation is performed according to animal weight change and death condition.

The tumor volume and animal body weight results were expressed as Mean ± SEM (mean standard error). All data were analyzed using GraphPad Prism 8.0. The differences in tumor volume among each group during the entire experiment were statistically analyzed through two-way ANOVA and Fisher's LSD, with p < 0.05 was considered statistically significant.

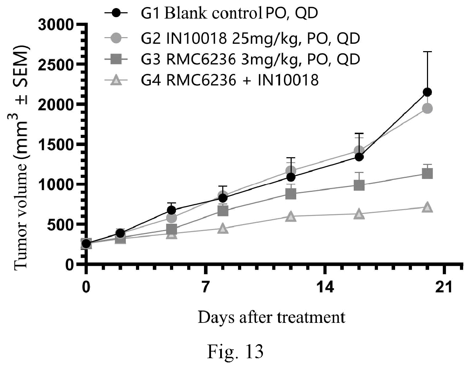

The tumor growth was observed every day, and the tumor volume, TGI value, and statistical analysis for each group are shown in Table 24. The tumor volume for each group at different time points is shown in Fig. 13.

**Table 24:**

| **Group** | **Tumor volume (day 0) (mm³)¹** | **Tumor volume (day 20) (mm³)** | **TGIₜᵥ (%)¹** | **P-value²** | **P-value³** |
|---|---|---|---|---|---|
| Blank control group | 260.0±42.1 | 2151.7±506.8 | / | / | / |
| IN10018 | 258.0±34.4 | 1945.7±174.8 | 10.8 | 0.2818 | <0.0001 |
| RMC6236 | 263.0±45.5 | 1135.4±118.3 | 53.9 | <0.0001 | 0.0003 |
| RMC6236 + IN10018 | 258.4±25.0 | 713.2±53.8 | 76.0 | <0.0001 | / |

| | | | | | |
|---|---|---|---|---|---|
| Note: 1. Mean ± SEM; 2. ****: p<0.0001, vs. blank control group, Two-way ANOVA; 3. ***: p<0.001, ****: p<0.0001, vs. RMC6236 + IN10018 group, Two-way ANOVA. | | | | | |

No abnormalities were found in the body weight of any of the experimental animals. The trend of body weight change in the treatment group was consistent with that in the vehicle control group. See Table 25 for details. These results indicate that the animal is well tolerated for treatment.

**Table 25:**

| **Group** | **Survival¹** | **Weight (Day 0) (g)²** | **Weight (Day 20) (g)²** | **Weight change rate (%)³** |
|---|---|---|---|---|
| Blank control group | 3/3 | 21.7±0.7 | 24.8±0.8 | 14.7 |
| IN10018 25 mg/kg | 2/3 | 21.7±0.3 | 23.1±0.7 | 7.5 |
| RMC6236 3/6 mg/kg | 3/3 | 22.2±0.4 | 23.9±1.1 | 7.5 |
| RMC6236 + IN10018 | 3/3 | 22.0±0.5 | 23.9±0.8 | 8.5 |

| | | | | |
|---|---|---|---|---|
| Note: 1. Number of animals surviving on day 12 / Number of animals surviving on day 0; 2. Mean ± SEM; 3. Weight change rate = (W₂₀-W₀) / W₀*100%. | | | | |

Compared with the blank control group, the IN10018 group, RMC6236 group, and RMC6236+IN10018 group showed significant differences, indicating that the above-mentioned monotherapy and combination therapy have significant anti-tumor effects, and the RMC6236+IN10018 group exhibited better anti-tumor activity.

All references mentioned in the present disclosure are incorporated herein by reference in their entirety, as if each reference were listed separately. It should be understood that various alterations or modifications to the present disclosure can be made by those skilled in the art after reading the present disclosure, and these equivalent forms also fall within the scope defined by the appended claims.

## Claims

1. Use of a FAK inhibitor and a Pan-RAS inhibitor in the manufacture of a medicament for treating a tumor in a subject.

2. Use of a FAK inhibitor in the manufacture of a medicament for use in combination with a Pan-RAS inhibitor to treat a tumor in a subject.

3. Use of a Pan-RAS inhibitor in the manufacture of a medicament for use in combination with a FAK inhibitor to treat a tumor in a subject.

4. A pharmaceutical combination product of a FAK inhibitor and a Pan-RAS inhibitor for treating a tumor in a subject.

5. A method for treating a tumor, the method comprising administering a therapeutically effective amount of a FAK inhibitor and a Pan-RAS inhibitor to a subject in need thereof.

6. A kit or pharmaceutical composition for treating a tumor, comprising:
(a) a FAK inhibitor; and
(b) a Pan-RAS inhibitor.

7. The use, pharmaceutical combination product, method, kit or pharmaceutical composition of any one of claims 1-6, wherein the FAK inhibitor and the Pan-RAS inhibitor are administered simultaneously or sequentially.

8. The use, pharmaceutical combination product, method, kit or pharmaceutical composition of any one of claims 1-7, wherein the FAK inhibitor is IN10018, Defactinib, AMP945, or a deuterated compound or pharmaceutically acceptable salt thereof; alternatively, IN10018, Defactinib, AMP945, deuterated compound 1 of Defactinib, deuterated compound 2 of Defactinib, or a pharmaceutically acceptable salt thereof; alternatively, IN10018, Defactinib, AMP945, or a pharmaceutically acceptable salt thereof; more alternatively, IN10018 or a pharmaceutically acceptable salt thereof.

9. The use, pharmaceutical combination product, method, kit or pharmaceutical composition of any one of claims 1-8, wherein the Pan-RAS inhibitor is RMC6236, Pan-RAS-IN-1, or ADT-007, or a pharmaceutically acceptable salt thereof; alternatively RMC6236.

10. The use, pharmaceutical combination product, method, kit or pharmaceutical composition of any one of claims 1-9, wherein the tumor is a tumor with RAS mutation; alternatively, wherein the RAS mutation is KRAS G12V, KRAS G12C, or KRAS G12D, alternatively KRAS G12V or KRAS G12C.

11. The use, pharmaceutical combination product, method, kit or pharmaceutical composition of any one of claims 1-9, wherein the tumor is bladder cancer, breast cancer, cervical cancer, colon cancer (including colorectal cancer), esophageal cancer, esophageal squamous cell carcinoma, head and neck cancer, liver cancer, lung cancer (including small cell lung cancer and non-small cell lung cancer), melanoma, myeloma, rhabdomyosarcoma, inflammatory myofibroblastoma, neuroblastoma, pancreatic cancer, prostate cancer, kidney cancer, renal cell carcinoma, fibrosarcoma (including osteosarcoma), skin cancer (including squamous cell carcinoma), gastric cancer, testicular cancer, thyroid cancer, uterine cancer, mesothelioma, bile duct carcinoma, leiomyosarcoma, liposarcoma, nasopharyngeal carcinoma, neuroendocrine carcinoma, ovarian cancer, salivary gland cancer, metastatic tumors caused by spindle cell carcinoma, anaplastic large cell lymphoma, undifferentiated thyroid carcinoma, non-Hodgkin's lymphoma, Hodgkin's lymphoma, glioma, or hematologic malignancies such as acute myeloid leukemia (AML), acute lymphoblastic leukemia (ALL), diffuse large B-cell lymphoma (DLBCL), follicular lymphoma (FL), chronic lymphocytic leukemia (CLL), or chronic myeloid leukemia (CML); alternatively, the tumor is fibrosarcoma, gastric cancer, lung cancer, breast cancer, glioma, esophageal cancer, pancreatic cancer, head and neck cancer, ovarian cancer, or colon cancer (including colorectal cancer); more alternatively, the tumor is colon cancer (including colorectal cancer), lung cancer, pancreatic cancer, or fibrosarcoma; even more alternatively, the tumor is colon cancer (including colorectal cancer) or fibrosarcoma.

12. The use, pharmaceutical combination product, method, kit or pharmaceutical composition of any one of claims 1-11, further comprising other therapeutic agents.

13. The use, pharmaceutical combination product, method, kit or pharmaceutical composition of any one of claims 12, wherein the other therapeutic agent is an immune checkpoint inhibitor.

14. The use, pharmaceutical combination product, method, kit or pharmaceutical composition of any one of claims 13, wherein the immune checkpoint inhibitor is a PD-1 or PD-L1 inhibitor, especially a PD-1 or PD-L1 antibody inhibitor.
